# EUROPEAN PATENT APPLICATION

(11) **EP 2 319 825 A1**
(43) Date of publication of application: **11.05.2011**
(21) Application number: 09797537.9
(22) Date of filing: 17.07.2009
(51) Int. Cl.: C07C 211/36, C07C 271/24, C07C 233/41, C07B 53/00

(54) **ENZYMATIC SYNTHESIS OF ENANTIOMERICALLY ENRICHED DERIVATIVES OF CIS- AND TRANS-CYCLOPENTANE-1,2-DIAMINES**

(30) Priority: 17.07.2008 ES 200802138
(71) Applicant: EntreChem, S.L., 33006 Oviedo Asturias (ES); Universidad De Oviedo, 33003 Oviedo (ES)
(72) Inventor: GONZÁLEZ SABÍN, Javier, E-33006 Oviedo - Asturias (ES); MORÍS VARAS, Francisco, E-33006 Oviedo - Asturias (ES); PEÑA GONZÁLEZ, Carmen, E-33006 Oviedo - Asturias (ES); QUIJADA SALDAÑA, Francisco Javier, E-33006 Oviedo - Asturias (ES); REBOLLEDO VICENTE, Francisca, E-33006 Oviedo - Asturias (ES); GOTOR SANTAMARÍA, Vicente, E-33006 Oviedo - Asturias (ES)
(74) Representative: ABG Patentes, S.L.
(86) International application number: PCT/ES2009/070293
(87) International publication number: WO 2010/007202

(57) **Abstract**

**SUMMARY**

The present invention relates to the enzymatic resolution of *cis-*and *trans-*cyclopentane-1,2-diamine derivatives to obtain enantiomerically pure or enriched compounds, a method for the preparation of the starting materials of that procedure, the products of this procedure and its transformation into products of commercial interest.

## Description

### FIELD OF INVENTION

The present invention relates to the enzymatic resolution of derivatives of *cis-*and trans-cyclopentane-1,2-diamines to obtain enriched or enantiomerically pure compounds, a method for the preparation of the starting materials of such procedure, the products of such procedure and its transformation into products of commercial interest.

### STATE OF THE ART

1,2-diamino function is present in the structure of a great variety of natural products such as biotin (vitamin H), the alkaloid slaframine or balanol, an inhibitor of protein kinase C. In addition, therapeutic properties of numerous optically active synthetic 1,2-diamines have been exploited in various fields of medicinal chemistry. Moreover, vicinal diamines are also compounds of great interest in coordination chemistry and asymmetric catalysis by acting as ligands and chiral auxiliaries, resolving agents and precursors of receptors.

Historically, *trans-*Cyclohexane-1,2-diamine diamine is perhaps the most used diamine for the synthesis of receptors and ligands. Thus, it has contributed decisively to the spectacular growth experienced by asymmetric catalysis in the last decade, being the structural key of some of the best known ligands such as salen ligands or Trost's ligand (Chem. Rev. 1997, 97, 3161). It has undoubtedly played in its favor the fact that this diamine is commercialized in enantiomerically pure form, since it is a nylon industry byproduct, easily crystallized with tartaric acid in diastereo- and enantiomerically pure form.

On the contrary, applications of its homologous optically active *trans-*cyclopentane-1,2-diamine remain largely unexplored due to the complexity and inefficiency of the reported synthesis. Moreover, in this case, failure of classic crystallization with optically active acids leads to enantiopure diamine only after several recrystallization cycles and in very low yield. In the case of the *cis* isomer of cyclopentane-1,2-diamine, the molecule is a meso form. The only optically active derivatives listed in the literature are obtained through an non-efficient chemoenzymatic synthesis.

Recently, peptide nucleic acids (PNA) incorporating the *cis-*and *trans-*cyclopentane-1,2-diamine unit have been described, and reportedly show excellent properties for the selective detection of DNA and RNA sequences [(1) J. Org. Chem. 2004, 69, 5725, (2) J. Am. Chem.Soc. 2004, 126, 15067]. In addition, there are theoretical evidences about the great potential of these diamines as chiral ligands in asymmetric catalysis (Angew. Chem. Int. Ed. 2001, 40, 4289).

Synthetic strategies described in the literature for the preparation of racemic *trans-*cyclopentane-1 ,2-diamine, are scarce and, in general, require tedious procedures, drastic reaction conditions and handling of hazardous intermediates. For example, the pioneering synthesis, described in the publication Z. Anorg. Chem., 1928, 175, 161, involved the difficult reduction of the dioxime of cyclopentane-1,2-dione in the last step. Moreover, as evidenced years later, the resulting product was a mixture of *cis*/trans isomers. Subsequently various modifications to the original synthesis were carried out, but the process continued to be impractical [(1) J. Am. Chem. Soc., 1951, 73, 1199, (2) J. Inorg. Nucl. Chem., 1971, 33, 3463].

Several years later WO 9717356 describes a three-step synthesis starting from *trans-*cyclopentane-1,2-diol (Figure 1). Thus, racemic *trans-*cyclopentane-1,2-diamine is easily obtained although it involves the hydrogenation of a explosive diazide.

Regarding *trans-*cyclopentane-1,2-diamine in enantiomerically pure form, state of the art in the literature is even scarcer.

It has been described in Org. Lett. 2002, 4, 3627 the sequential kinetic resolution of racemic *trans-*cyclopentane-1,2-diamine to obtain its enantiomers by means of a double aminolysis reaction with dimethyl malonate catalyzed by lipase B from *Candida antarctica.* Enantioselectivity of the process is very high although the method suffers from the need to prepare and manipulate previously free racemic diamine, which is extremely unstable and oxidizes even under inert atmosphere. Subsequently, it has been performed the enzymatic resolution of several precursor diamines of *trans-*cyclopentane-1,2-diamine in which one of the primary amino groups is masked as a tertiary amine with various protective groups. For example, J. Org. Chem. 2007, 72, 1309 describes the resolution of derivatives of *trans-*cyclopentane-1,2-diamine in which one of the amino groups is masked in the form of various tertiary amines.

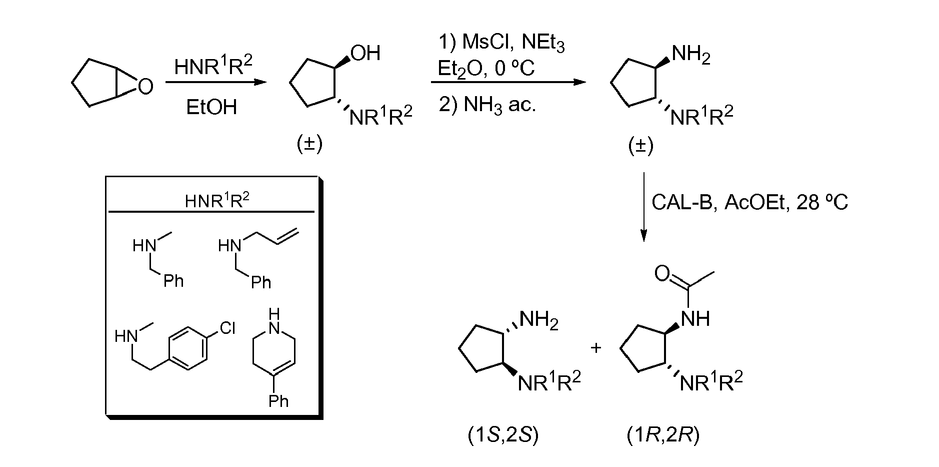

In Tetrahedron: Asymm. 2008, 19, 751-755 it is described a similar procedure, but where an amino group is masked as a diallylamino group.

It has been recently published (J. Org. Chem. 2006, 71, 8655) the resolution of racemic *tert-*butyl (±)-*trans-N-*(2-aminocyclopentyl)carbamate by means of fractional crystallization with optically active 10-camphorsulfonic acid. Previously, *tert-*butyl (±)-*trans-N-*(2-aminocyclopentyl)carbamate is prepared in four steps with a yield of 64% and, below, the enantiopure compound is obtained with a yield of 51% only after several cycles of recrystallization.

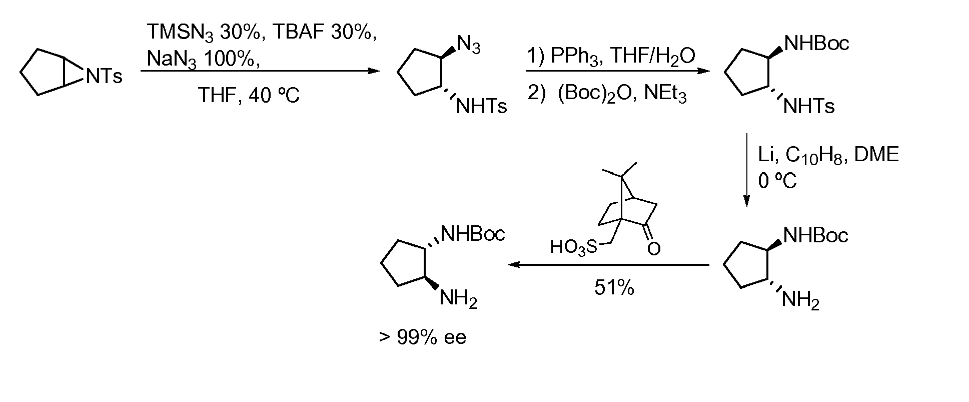

Regarding racemic *cis-*cyclopentane-1,2-diamine, one of the few methods of preparation is reported in the publication Tetrahedron 2002, 58, 1131. Starting from cyclopentene, Sharpless oxidation affords *cis-*cyclopentane-1,2-diol, which is transformed into the corresponding *cis-*diazide by mesylation of the hydroxyl groups. Finally, palladium-catalyzed hydrogenation enables the isolation of *cis-*cyclopentane-1,2-diamine as dichlorhydrate salt.

Regarding optically active derivatives of *cis-*cyclopentane-1,2-diamine, the only description reported is found in J. Org. Chem. 2004, 69, 5725. The synthetic strategy consists of the kinetic resolution of racemic *trans-*2-azidocyclopentyl butyrate by means of enzymatic hydrolysis and its further transformation into the optically active *tert-*butyl *cis-N-*(2-aminocyclopentyl) carbamate. However, the resolution process is inefficient and requires two cycles to obtain the enantiomerically pure *trans-*2-azidocyclopentanol. Finally, each one of these enantiomers provides access to the corresponding enantiomers of *tert-*butyl *cis-N-*(2-aminocyclopentyl) carbamate after four steps in 70%yield.

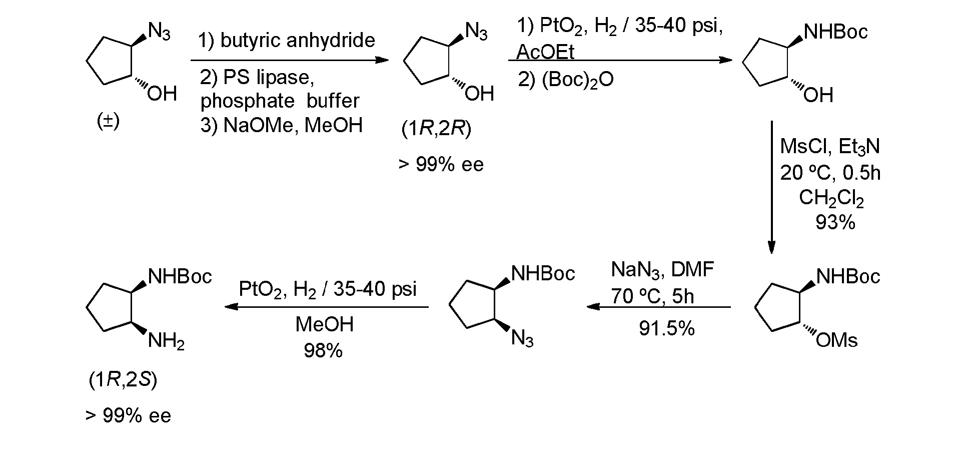

Taking into account that the components of a racemic mixture can produce very different biological effects, there is a need to access to *cis* and *trans-*cyclopentane-1,2-diamine and their derivatives, in particular access to monosubstituted derivatives in enantiopure or enantiomerically enriched form.

Therefore, there is a need to develop a procedure for obtaining enantiomerically pure or enantiomerically enriched *cis-*and *trans-*cyclopentane-1,2-diamine, which overcomes the problems described above and allows an efficient obtention of the diamine and its derivatives.

### BRIEF DESCRIPITION OF THE INVENTION

Inventors have found that the resolution of *cis-* and *trans-*cyclopentane-1,2-diamine carbamates in the presence of hydrolases provides a surprisingly efficient method for obtaining the corresponding enantiomers in high enantiomeric excess, and the subsequent transformation into commercially interesting compounds, procedures which constitutes aspects of the present invention as described below.

Therefore, according to a first aspect, the present application relates to a process for obtaining a mixture comprising a compound of formula (I) enantiomerically enriched, or salts thereof, and a compound of formula (II) enantiomer enriched, or salts thereof, or to obtain a mixture comprising an enantiomer of the compound of formula (I) enantiomerically enriched, or salts thereof, and the enantiomer of the compound of formula (II) enantiomerically enriched, or salts thereof, 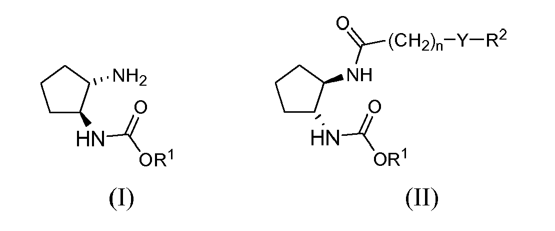 Where
R¹ is selected from the group consisting of substituted or unsubstituted alkyl, substituted or unsubstituted alkenyl and substituted or unsubstituted arylalkyl;
R² is selected from the group consisting of hydrogen, substituted or unsubstituted alkyl, haloalkyl and unsubstituted or mono-, di-or trisubstituted phenyl with identical or different substituents selected from the group consisting of halogen, amino, hydroxyl, C₁-C₄ alkyl, C₁-C₄ alkoxy, phenyl and phenoxyl;
n represents the numbers 0, 1, 2 or 3, and
Y represents a bond, oxygen, sulfur, or a -NR⁸ group where R⁸ represents hydrogen or C₁-C₄ alkyl group;
which comprises reacting a mixture comprising the compound of formula (I) and its enantiomer, or its salts, with an acylating agent in the presence of an enzyme.

Likewise, the invention also relates to a process for obtaining a mixture comprising a compound of formula (IX) enantiomerically enriched, or salts thereof, and a compound of formula (X) enantiomerically enriched, or salts thereof, or to obtain a mixture comprising an enantiomer of compound of formula (IX) enantiomerically enriched, or salts thereof, and the enantiomer of compound of formula (X) enantiomerically enriched, or salts thereof, 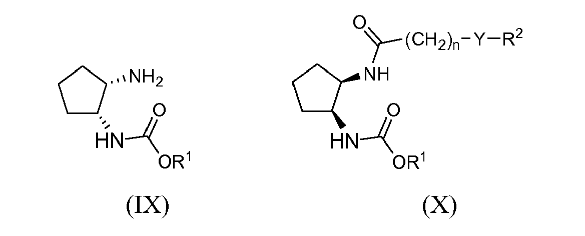 where
R¹, R², n and Y are as defined above;
which comprises reacting a mixture comprising the compound of formula (IX) and its enantiomer, or its salts, with an acylating agent in the presence of an enzyme.

Therefore, the process of the present invention can be considered a method of resolving a mixture of a compound of formula (I), or its salts, and its enantiomer, or a method of resolving a mixture of a compound of formula (IX), or its salts and its enantiomer. The selective acylation of the enantiomer of compound of formula (I) or compound of formula (IX) by the action of the hydrolase present in the reaction medium allows to obtain the corresponding compound of formula (II) or formula (X), respectively, enantiomerically enriched, together with compound of formula (I) or formula (IX). The enantiomeric excesses obtained are excellent. The procedure for the preparation of enantiomerically enriched compound of formula (I) or formula (IX) as described above, their enantiomers or salts thereof, are thus additional aspects of the present invention.

Therefore, the process of the present invention, provides a mixture comprising a compound of formula (I) enantiomerically enriched, or salts thereof, and a compound of formula (II) enantiomerically enriched, or salts thereof, or a mixture comprising the enantiomer of compound of formula (I) enantiomerically enriched, or salts thereof, and the enantiomer of compound of formula (II) enantiomerically enriched, or salts thereof; or a mixture comprising a compound of formula (IX) enantiomerically enriched, or salts thereof, and a compound of formula (X) enantiomerically enriched, or salts thereof, or a mixture comprising an enantiomer of the compound of formula (IX) enantiomerically enriched, or salts thereof, and the enantiomer of compound of formula (X) enantiomerically enriched, or salts thereof These mixtures obtained through the procedure developed by the inventors, are also an object of the present invention.

Once obtained the mixtures of compounds of formula (I) and (II) or mixtures of compounds of formula (IX) and (X), they can be separated by acid-base extraction, recrystallization or a combination of both, to obtain the corresponding compounds of formula (I) or formula (IX) enantiomerically enriched, or salts thereof, or its enantiomers and compounds of formula (II) or formula (X) enantiomerically enriched, or salts thereof, or their enantiomers, which constitutes an additional aspect of the present invention. The compounds of formula (II) or formula (X) enantiomerically enriched, or its enantiomers or salts thereof isolated also constitute an object of the present invention. To the best knowledge of the inventors, have not been described so far, and the fact that both nitrogens are protected by protecting groups which can be cleaved under similar conditions, can reduce the number of steps of deprotection to obtain trans-cyclopentane-1,2-diamine, and its derivatives, enantiomerically enriched.

The choice of the carbamate group allows to obtain directly and efficiently the enantiomerically enriched monoalkylated derivatives of *trans-*cyclopentane-1,2-diamine, versatile intermediates for the synthesis of biologically interesting compounds and chiral ligands.

Therefore, a further aspect of the present invention relates to a procedure for obtaining enantiomerically enriched *trans-*cyclopentane-1,2-diamine or *meso cis-*1,2-cyclopentane-1,2-diamine, or salts thereof which comprise the following steps:
a) reacting a mixture of the compound of formula (I) or the compound of formula (IX) and its enantiomer, or its salts with an acylating agent in the presence of a hydrolase;
b) separating the mixture obtained in the previous step into compounds (II) and (I) or compounds of formula (X) and (IX), enantiomerically enriched, or their enantiomers, preferably by acid/base extraction, recrystallization or a combination of both; and
c) cleaving the carbamate group of the compound of formula (I) or the compound of formula (IX); and/or sequential or simultaneous split of the groups -(C=O)-(CH₂)ₙ-Y-R² and carbamate of this compound of formula (II) or compound of formula (X), preferably by acid or basic hydrolysis.

A further aspect of the present invention is the use of a mixture comprising a compound of formula (I) and its enantiomer or salts thereof to obtain enantiomerically enriched *trans-*cyclopentane-1,2-diamine, or salts thereof.

A further aspect of the present invention is a procedure for obtaining a compound of formula (VI) or its enantiomer, enantiomerically enriched 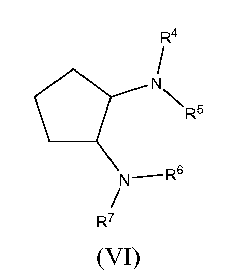 where
R⁴, R⁵, R⁶ and R⁷ are independently selected from hydrogen or a radical selected from the group consisting of alkyl, cycloalkyl, aromatic or non aromatic heterocycle, hydroxyalkyl, halogenated alkyl, alkoxyalkyl, alkenyl, alkynyl, aryl, alkylaryl, COOR, carbonate, -COOH, -COH, -COR, -(C=O)NH₂, -(C=O) NRₐR_{b}, a silyl group, -S(=O)R or a combination of them; and where R and R^{a} and R^{b} independently selected from each other, represent a C₁-C₈ alkyl group;
which comprise the preparation of enantiomerically enriched *trans-*cyclopentane-1,2-diamine or *meso cis-*cyclopentane-1,2-diamine, or its salts, according to the procedure developed by the inventors; and the functionalization of amino groups for the introduction of the groups R⁴, R⁵, R⁶ and R⁷.

The procedures for functionalization of amines or acid salts of amine are known to those skilled in the art (for example, see Smith, M.B.; March J. March 's Advanced Organic Chemistry; John Wiley & Sons, 5th Edition, 2001).

A further aspect of the present invention is the use of a mixture comprising a compound of formula (I) and its enantiomer or salts thereof, or a compound of formula (IX) and/or its enantiomers or salts thereof, to obtain a compound of formula (VI) enantiomerically enriched, or its enantiomer, or salts thereof.

According to a further aspect, the present invention relates to a mixture of the enantiomers of benzyl *trans-*(2-aminocyclopentyl) carbamate (compound 6) or a mixture of the enantiomers of *tert-*butyl *cis-*(2-aminocyclopentyl) carbamate (Compound 15)" 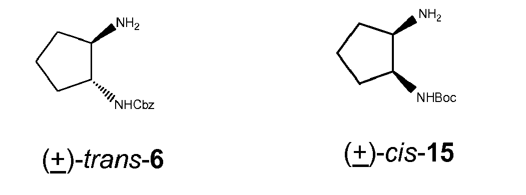 or salts thereof, starting material of the resolution procedure developed by the inventors and a compound of formula (6) or (15) or its enantiomers.

All starting materials and reagents are commercially available, allowing the procedure to be applied to industrial scale.

Inventors have also developed a method for preparing mixtures of a compound of formula (I) and its enantiomer. Therefore, according to a further aspect, the present invention relates to a process for preparing a racemic mixture of a compound of formula (I) and its enantiomer, or its salts, which comprise removing allyl groups, preferably in presence of palladium, from the racemic mixture of a compound of formula (VII) and its enantiomer 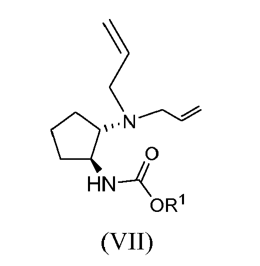 where R¹ is selected from substituted or unsubstituted alkyl, substituted or unsubstituted alkenyl and substituted or unsubstituted arylalkyl.

Racemic mixtures of compounds of formula (VII) and its enantiomers, except those in which R¹ represents *tert-*butyl, or salts thereof, useful intermediates for the preparation of starting materials, are also an aspect of the present invention.

### DETAILED DESCRIPTION OF THE INVENTION

### Definitions

"Enantiomerically enriched" in the present invention applied to a mixture of enantiomers is referred to a mixture of enantiomers, for example a mixture comprising a compound of formula (I) and its enantiomer, wherein one of the enantiomers is present in excess to the other enantiomer. Therefore, the enantiomerically enriched mixtures have an enantiomeric excess higher than 0% with respect to one of its enantiomers, preferably above 20%, preferably above 40%, preferably above 70%, more preferably greater than 80%, more preferably above 90% and more preferably greater than 95%.

An "enantiomerically pure" or "enantiopure" compound for the purposes of the present invention relates to a mixture of two enantiómeros having an enantiomeric excess above 95%, preferably greater than 98%, more preferably greater than 99%, more preferably higher than 99.5%.

The term "resolution" in the present invention relates to a procedure that increases the enantiomeric excess of a mixture of two enantiomers. For example, resolution of a racemic mixture refers to a procedure starting from an equimolar mixture of two enantiomers, and producing a mixture in which one of the enantiomers is in greater proportion than the other. Similarly, it is possible to start from a mixture of two enantiomers in which one of the enantiomers is found in greater proportion than the other, and as a result of the resolution process, the ratio between the two enantiomers is different than the original.

"Alkyl" refers to a radical of saturated linear or branched hydrocarbon chain consisting of carbon and hydrogen atoms, and that in the absence of additional indications has 1-12 carbon atoms, preferably from one to eight carbon atoms, and that is attached to the rest of the molecule by a single bond, for example, methyl, ethyl, n-propyl, *i*-propyl, *n*-butyl, *t*-butyl, *n*-pentyl, etc. Alkyl radicals may be optionally substituted by one or more substituents, preferably 1 to 5, more preferably 1 or 2, such as halogen, hydroxyl, alkoxy such as *O*-propyl or *O*-benzyl, benzoate, carboxyl, cyano, carbonyl, acyl, alkoxycarbonyl, amino, imino, nitro, mercapto and alkylthio.

"Cycloalkyl" refers to fraction of alicyclic saturated monocyclic hydrocarbon, linked to the rest of the molecule by a single bond, and which is optionally substituted with 1, 2 or 3 residues of monovalent saturated hydrocarbon, linear or branched, such as cyclopropyl, cyclobutyl, cyclopentyl, cyclohexyl, cycloheptyl, methylcyclohexyl, dimethylcyclohexyl and the like.

"Heterocyclic aromatic or non-aromatic" refers to a stable cyclic system from 3 to 15 members composed of carbon atoms and from one to five heteroatoms selected from the group consisting of nitrogen, oxygen and sulfur, preferably a ring of 4 to 8 members with one or more heteroatoms, more preferably a ring of 5 or 6 members with one or more heteroatoms. For the purposes of this invention, the heterocyclic ring system may be a monocyclic, bicyclic or tricyclic cyclic ring system, which may include condensed ring systems; and the atoms of nitrogen, carbon or sulfur may optionally be oxidized in the heterocyclic radical; the nitrogen atom can be optionally quatemized, and the heterocyclic radical may be partially or completely saturated or be aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.

"Hydroxyalkyl" refers to an alkyl residue as defined above, containing 1, 2 or 3 hydroxy groups such as -CH₂OH, -CH₂CH₂OH, -CH(OH)CH₃, -CH₂CH₂CH₂OH, - CH(OH)CH(OH)CH₂OH, and the like.

"Halogenated alkyl" or "haloalkyl" means an alkyl residue as defined above where at least one of the positions is replaced by a halogen, ie, a substituent selected from the group consisting of -F, -Cl, -Br and -I. Haloalkyl groups may be substituted in all positions by the same or different halogen. For example, if all positions are replaced by halogen groups, it is defined as "perhalogenated"alkyl; if all positions are substituted by fluorine atoms, it is defined "perfluorinated alkyl"; or if all positions are replaced by chlorine atoms, it is defined as "perchlorated" alkyl. Alternatively, the haloalkyl group may be substituted in some of their positions, for example, 1, 2, 3 or more positions. Non-limiting examples of haloalkyl groups are CH₂F, -CH₂Cl, -CH₂Br,-CHF₂, -CF₃, - CCl₃, -CF₂CF₃ and the like.

"Alkoxy refers to a radical of the formula -O-alkyl, where alkyl is defined above, for example, methoxy, ethoxy, propoxy, etc.

"Alkenyl" refers to a radical of linear or branched hydrocarbon chain consisting of carbon and hydrogen atoms, containing at least one unsaturation, having 2-12 carbon atoms, preferably from two to eight carbon atoms, and attached to the rest of the molecule by a single bond, for example, ethenyl, *n*-propenyl, *i*-propenyl, prop-2-enyl (allyl), *n*-butenyl, *n*-pentenyl, etc. Alkenyl radicals may be optionally substituted by one or more substituents such as halogen, hydroxyl, alkoxy such as *O*-propyl or *O*-benzyl, benzoate, carboxyl, cyano, carbonyl, acyl, alkoxycarbonyl, amino, imino, nitro, mercapto and alkylthio.

"Alkynyl" means a fraction of monovalent unsaturated aliphatic hydrocarbon, linear or branched, having 2-12 carbon atoms, preferably from two to eight carbon atoms, and containing 1, 2 or 3 carbon-carbon triple bonds conjugated or unconjugated, such as-CCH, -CH₂CCH, CCCH₃, CH₂CCCH₃, and the like.

"Aryl" refers to a monocyclic aromatic hydrocarbon radical, bicyclic or tricyclic of 6 to 14 members, such as phenyl, naphthyl, anthracyl or 9H-fluorenyl (fluorenyl). The aryl radical may be optionally substituted by one or more substituents such as hydroxyl, mercapto, halogen, alkyl, phenyl, alkoxy, haloalkyl, nitro, cyano, dialkylamino, aminoalkyl, acyl and alkoxycarbonyl.

"Arylalkyl" or "aralkyl" refers to an aryl group attached to an alkyl group such as benzyl, phenethyl or fluorenylethenyl.

"Silyl", commonly referred to as trialkylsilyl, are groups attached to the rest of the molecule through a silicon atom, which is substituted by three alkyl groups equal or different from each other. Illustrative examples may be the trimethylsilyl (TMS), triethylsilyl, *tert-*butyldimethylsilyl ("TBDMS"), *tert-*butyldiphenylsilyl, triisopropylsilyl, diethylisopropylsilyl, texyldimethylsilyl ether, triphenylsilyl, or di-*tert-*butylmethylsilyl.

References in this document to "substituted groups" in the compounds of the present invention relate to the specified group which may be substituted in one, two, three or more available positions by one or more suitable groups, for example, halogen such as fluorine , chlorine, bromine and iodine; cyano; hydroxyl; nitro; azido; alkoxy groups having one or more links of oxygen and from 1 to about 11 carbon atoms or from 1 to about 6 carbon atoms; aryloxy such as phenoxyl; alkylthio groups including those groups which have one or more thioether bonds and from 1 to about 12 carbon atoms or from 1 to about 6 carbon atoms, amino groups; aminoalkyl groups such as groups having one or more N atoms and from 1 to about 12 carbon atoms or from 1 to about 6 carbon atoms; aryl having 6 or more carbons, particularly phenyl or naphthyl and aralkyl such as benzyl. Unless otherwise indicated, an optionally substituted group may have a substituent in each substitutable position of the group, and each substitution is independent of the other.

### Procedure of resolution

The procedure of the present invention comprises an enzymatic resolution of a mixture of a compound of formula (I) or (IX) and their respective enantiomers, or their salts. By action of the biocatalyst or enzyme, one enantiomer (either the compound of formula (I) or (IX), or their respective enantiomers) is selectively acylated, while the other enantiomer remains unreacted. It should be noted that the choice of enzyme can determine which of the two enantiomers is acylated. For the purpose of the present invention the compound of formula (I) or (IX), respectively, have been depicted as the compounds which is not acylated and remain intact. 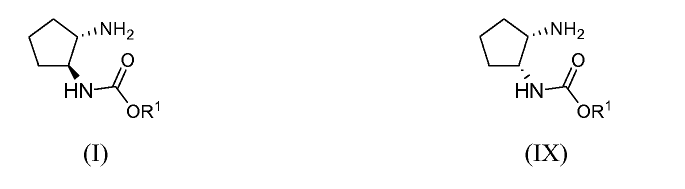 And it is depicted as acylated compound, the enantiomer of the compound of formula (I) or (IX), leading to the compound of formula (II) or (X), respectively. However, it is obvious to those skilled in the art that, depending on the selected enzyme, the reaction could proceed on the contrary, the enantiomer of the compound of formula (I) or (IX) remaining intact and the compound of formula (I) or (IX) being acylated, in which case, it would be obtained the enantiomer of the compound of formula (II) or the enantiomer of the compound of formula (X).

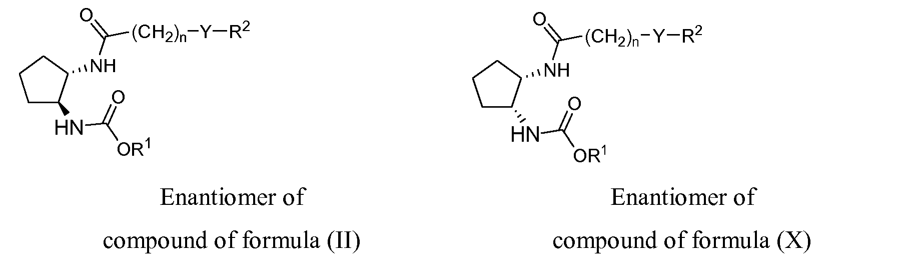

For example, in the resolution of the compound (±)-*trans*-5 (Scheme 1), the enzyme preferably catalyzes the acylation of the enantiomer (1*R*,2*R*)**-5** (a compound of formula (I)), leading to the product (1*R*,2*R*)**-7** (a compound of formula (II)), while the isomer (1*S*,2*S*)**-5** remains largely unreacted. The assignment of the absolute configuration of (1*S*,2*S*)**-5** was carried out by comparing the sign of its optical rotation with that published for this compound in the article J. Org. Chem. 2006, 71, 8655.

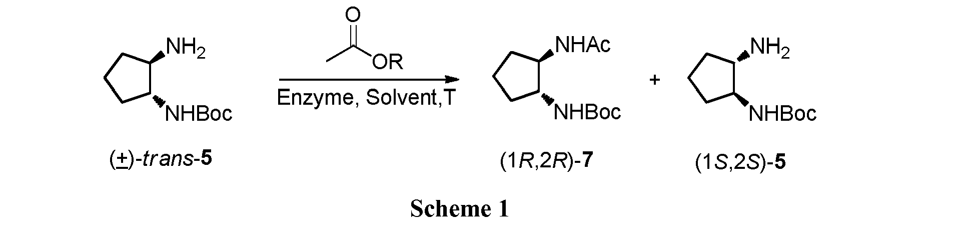

Similarly, in the resolution of compound (±)-*trans-***6** (a compound of formula (I)) (See Scheme 2), the enzyme preferably catalyzes the acylation of the enantiomer (1*R*,2*R*)-**6**, leading to the product (1*R*,2*R*)-**8** (a compound of formula (II)), while the isomer (1*S*, 2*S*)-**6** remains largely unreacted. To determine the absolute configuration of compounds (1*S*,2*S*)-**6** and (1*R*,2*R*)-**8** obtained, first an acid hydrolysis of (1*S*,2*S*)-**6** with 3N aqueous HCl is carried out to give dihydrochloride (1*S*,2*S*)-**10** (dihydrochloride of *trans* (1*S*,2*S*)-cyclopentane-1,2-diamine). Then the absolute configuration is assigned by comparing of its sign of optical rotation with that published in the article Acta. Chem. Scand. 1972, 26, 4019.

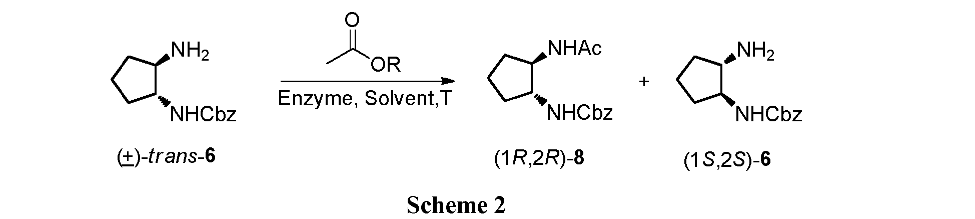

Therefore, in both exemplified resolution processes, acylation of the compound of formula (I) with (*R*,*R*) configuration is more favored than that of its enantiomer (*S,S*). However, as mentioned before, this preference may change depending on the structure of the compound of formula (I) and its enantiomer, or the enzyme used.

Similarly, in the resolution of compound *cis* (±)-15 (a compound of formula (IX)) (See Scheme 3), the enzyme preferably catalyzes the acylation of the enantiomer (1*S*,2*R*), resulting in the product (1*S*,2*R*)-**16** (a compound of formula (X)), while the isomer (1*R*, 2*S*)-**15** remains largely unreacted. The absolute configuration of both compounds were tentatively assigned by analogy with the previous examples and according to the prediction of Kazlauskas' rule (J. Org. Chem. 1991, 56, 2656), since in this case data of optical rotation of these compounds have not been reported in the literature.

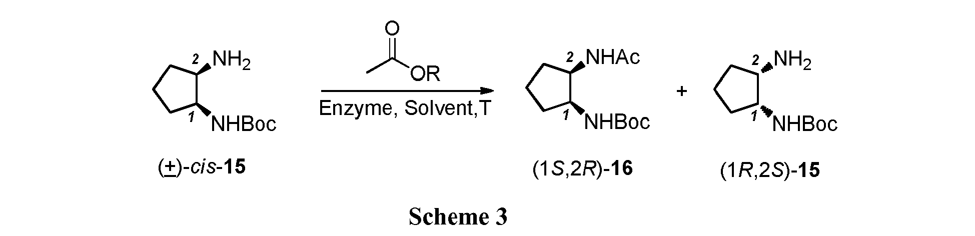

As the conversion of the resolution process increases, the preferred enantiomer is consumed, while the other remains practically intact. The specific value of conversion to which we must stop the reaction will depend on the enantioselectivity of each individual case and the requirements of optical purity of the products. When the reaction is sufficiently enantioselective, conversion of the reaction should be close to 50% (when the initial mixture of the compound of formula (I) or formula (IX) and its enantiomer is a racemic mixture) to get the maximum yield of enantiomerically enriched acylated product and remaining substrate. However, when the enantioselectivity is moderate, other conversion values may be preferable to ensure a sufficiently high value of enantiomeric excess of one of the components of the reaction mixture. For example, it is known that with increasing conversion, in a given reaction conditions, the enantiomeric excess of remaining substrate increases and the enantiomeric excess of product declines. The enantiomeric excess values are determined in accordance with the work described in the literature by Sih et al. (J. Am. Chem. Soc. 1982, 104, 7294). Once reached the desired conversion value the reaction is stopped - by filtration of the enzyme, for example- and the resulting compounds can be separated. This separation can be done by chromatography or by acid-base extraction, the latter being especially suitable because of its simplicity and effectiveness. Alternatively, the mixture can be subjected to appropriate modifications and then separate the resulting products. Therefore, according to a preferred embodiment, the method comprises a separation step of the compounds (II) and (I), or their enantiomers, or the compounds of formula (IX) and (X), or their resulting enantiomers. Preferably, the method comprises the separation by acid-base extraction, recrystallization or a combination thereof.

### Starting materials, preparation and synthetic intermediates

The starting materials of the resolution procedure, aspects of the present invention, are mixtures of compounds of formula (I) and its enantiomers, or compounds of formula (IX), or its enantiomers, preferably racemic mixtures, according to a preferred embodiment *tert-*butyl *trans-N-*(±)-(2-aminocylopentyl) carbamate (Compound (±)**-5**) or benzyl *trans-*(±)-*N-*(2-aminocylopentyl) carbamate (compound (±)**-6**). The scheme 4 shows as an example the complete sequence of preparation of the mixture of a compound of formula (I) and its enantiomer.

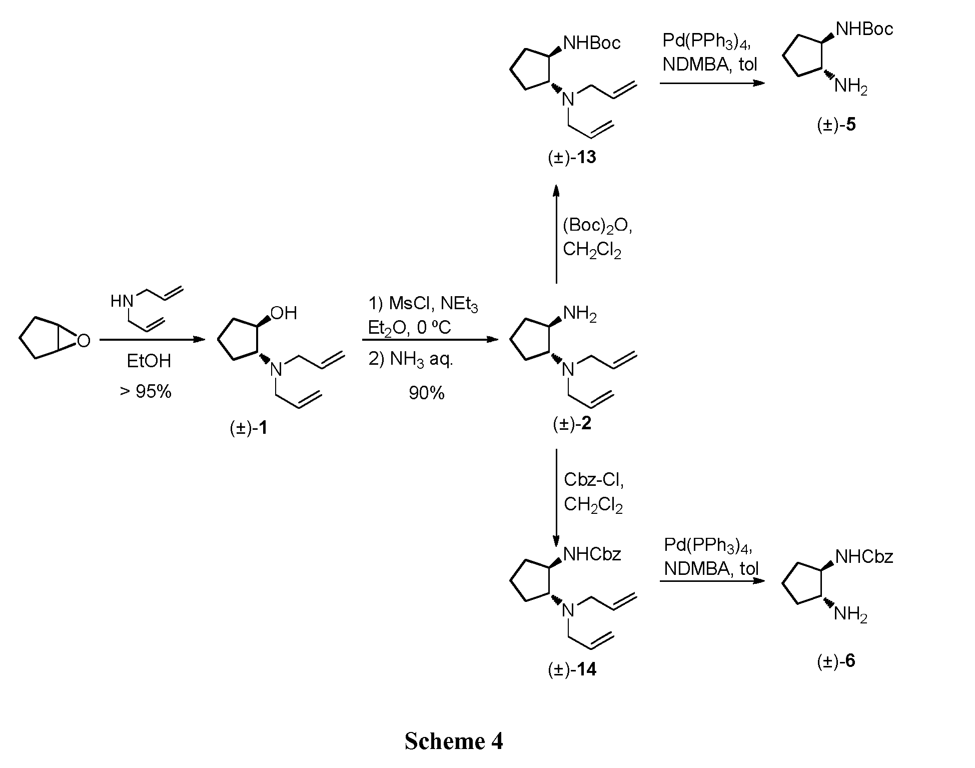

Compounds of formula (I) can be obtained from the racemic mixture of a compound of formula (VII) and its enantiomer (in the scheme 4 are shown compounds (±)-**13** and (±)-**14** as example) which, preferably, can be obtained by carbamoylation of *trans-*(±)-*N,N-*diallylcyclopentane-1 ,2-diamine (Compound (±)-**2** Scheme 4) in the presence of an agent of carbamoylation. Useful carbamoylation agents for the present invention are known to those skilled in the art (see, for example, Greene, T. W.; Wuts, P. G. M. Greene's Protective Groups in Organic Synthesis, John Wiley & Sons: Hoboken, 2007). For example with the corresponding anhydride with formula R¹OC(C=O)O(O=C)OR¹, or the corresponding acid halide of formula X(O=C)OR¹, wherein R¹ is as defined above and X is fluorine, chlorine, bromine or iodine, preferably chlorine, for example, Boc anhydride (*t*-BuOC(C=O)O(O=C)Ot-Bu) or benzyloxycarbonyl chloride (Cl(O=C)OCH₂phenyl), and further desallylation catalyzed by a complex of palladium and 1,3-dimethylbarbituric acid.

According to a preferred embodiment, R¹ in any of the compounds described in the present invention is selected from the group consisting of methyl, ethyl, *iso*-butyl, *tert-*butyl, allyl, benzyl, *p*-methoxybenzyl and 9-fluorenylethylene.

*trans-*(+)-*N,N-*diallylcyclopentane- 1 ,2-diamine can be obtained from inexpensive and commercially available cyclopentene oxide, according to the method described in Tetrahedron: Asymm. 2008, 19, 751-755. The opening of cyclopentene oxide with diallylamine provides the amino alcohol *trans-*(±)**-1**, which becomes *trans-*(±)-*N,N-*diallylcyclopentane-1,2-diamine (*trans-*(±)**-2**) by successive treatment with mesyl chloride and an aqueous solution of ammonia.

According to the procedure shown in scheme 4, one of the intermediates in the preparation of the mixture of compounds of formula (I) and its enantiomers, are the mixtures of a compound of formula (VII) and its enantiomer, or its salts 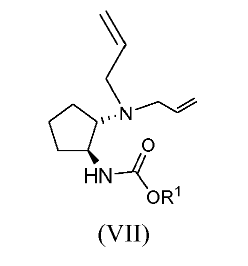 where R¹ is as defined above; more preferably R¹ is selected from *tert-*butyl and benzyl.

According to a preferred embodiment, said compounds of formula (VII) are the *tert-*butyl (±)-*N-*[2-(*N*'*,N*'*-*diallylamine) cyclopentyl] carbamate (compound (±)**-13**) or the benzyl (±)-*N-*[2-(*N*'*,N*'*-*diallylamine) cyclopentyl] carbamate (compound (±)**-14**).

In the case of the *cis* isomers of cyclopentane-1,2-diamine, the starting materials of the resolution procedure, are mixtures of compounds of formula (IX) and its enantiomers, preferably racemic mixtures, according to a preferred embodiment *tert-*butyl *cis-N-*(2-aminociclopentyl) carbamate (Compound (±)-15). The scheme 5 shows the complete sequence of preparation of the racemic mixture of the compound of formula 15, according to the protocol described in J. Org. Chem. 2004, 69, 5725, although in that case takes place with the respective compounds in enantiomerically pure form.

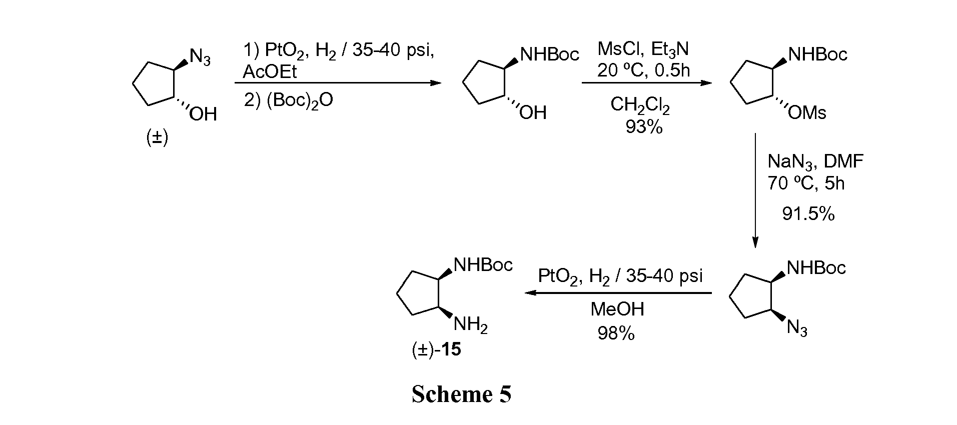

### Reaction conditions for the resolution

The process is carried out by dissolving the substrate in a suitable solvent and adding the enzyme and the acylating agent.

As reaction medium can be used an organic solvent. A suitable solvent can be an ether. However, other solvents such as toluene, hydrocarbons or alcohols can also be useful. According to a preferred embodiment the solvent is selected from ethyl acetate, dioxane, tetrahydrofuran, *tert-*butyl methyl ether, di-*iso*-propyl, diethyl ether, toluene, hexane, acetonitrile, acetone, propan-2-ol, 2-methylpropan-2-ol, chloroform and dichloromethane; more preferably, the solvent is ethyl acetate.

Preferably, the enzymes are hydrolases, both from animal and microbial origin, in pure or semipurified form, free or immobilized, preferably a lipase. Useful enzymes for the acylation can be found in Tetrahedron 2004, 60, 501; Chem. Soc. Rev. 2004, 33, 201, or Adv. Synth. Catal. 2006, 348, 797. According to a preferred embodiment, the enzyme is the *Candida antarctica lipase* (CALB), for example Novozyme SP435, commercially accessible. This lipase is available in different forms of immobilization on supports, preferably hydrophobic and mechanically resistant or acrylic resins, such as an epoxiacrylic resin activated with deca-octyl groups.

Therefore, according to a preferred embodiment, the enzyme is immobilized on a support, preferably by interfacial adsorption on hydrophobic supports and mechanically resistant. According to a particular embodiment, the lipase is immobilized on an epoxiacrylic resin activated with deca-octyl groups.

Useful acylating agents for the present invention are those which can act as substrates of the enzyme used resulting in the acetylation of the compound of formula (I) or compound of formula (IX), or its salts. According to a preferred embodiment, the acylating compound is a compound of formula (VIII) 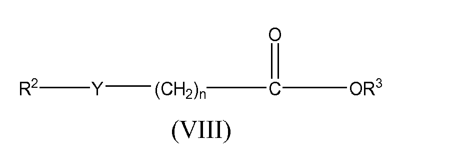 where
R², Y and n are as defined above; and
R³ is selected from the group consisting of substituted or unsubstituted alkyl and haloalkyl.

In one embodiment of the present invention, -(CH₂)ₙ-Y-R² in any of the compounds described herein represents methyl, methoxymethyl, chloromethyl and bromomethyl.

In one embodiment of the present invention, the acylating agent is selected from the group consisting of ethyl acetate, isopropyl acetate, methyl methoxyacetate, *iso-*propyl methoxyacetate, ethyl chloroacetate, ethyl bromoacetate, methyl chloroacetate, methyl bromoacetate, α-methylbenzyl acetate, α-methylbenzyl methoxyacetate, α-methylbenzyl chloroacetate and α-methylbenzyl bromoacetate; more preferably, the acylating agent is selected from ethyl acetate, methyl methoxyacetate, ethyl chloroacetate and α-methylbenzyl acetate; even more preferably ethyl acetate. In a particular embodiment, the solvent is ethyl acetate. Preferably, the acylating agent and solvent are the same, preferably ethyl acetate, which has clear advantages in terms of ease, cost and security, besides providing excellent enantiomeric excesses.

The reaction temperature will depend on the particular enzyme used, the acylating agent and the nature of the compounds of formula (I) and (IX) initially used. Generally it should be a temperature that allows to maintain the enzyme intact, without causing denaturalization. The reaction can be carried out between 5 and 60 °C, preferably between 10 and 60 °C, more preferably between 15 and 40° C.

### Products of the resolution process

Therefore, the product of the process of resolution developed by the inventors is a mixture comprising a compound of formula (I) enantiomerically enriched, or salts thereof, and a compound of formula (II) enantiomerically enriched, or salts thereof, or a mixture comprising an enantiomer of the compound of formula (I) enantiomerically enriched, or salts thereof, and the enantiomer of the compound of formula (II) enantiomerically enriched, or salts thereof. Similarly, in the case of *cis-*cyclopentane-1,2-diamine the product of the resolution procedure developed by the inventors is a mixture comprising a compound of formula (IX) enantiomerically enriched, or salts thereof, and a compound of formula (X) enantiomerically enriched, or salts thereof, or a mixture comprising the enantiomer of the compound of formula (IX) enantiomerically enriched, or salts thereof, and the enantiomer of the compound of formula (X) enantiomerically enriched, or salts thereof.

The separation of these mixtures by acid-base extraction, recrystallization or a combination of both, which is an additional aspect of the present invention, provides access to compounds of formula (II) or (X), respectively, enantiomerically enriched, or its enantiomers or salts thereof.

Preferred compounds of formula (II) are the *tert-*butyl (1*S*,2*S*) or the *tert-*butyl (1*R*,2*R*)-*N-*[2-(acetylamino)cyclopentyl)] carbamate or the benzyl (1*S*,2*S*) or the benzyl (1*R*,2*R*)-*N-*[2-(acetylamino)cyclopentyl)] carbamate.

Preferred compounds of formula (X) are the (1*S*,2*R*) or the *tert-*butyl (1*R*,2*S*)-N [2-(acetylamino)cyclopentyl)] carbamate.

In other known methods is necessary to derivatize the products of the resolution before separating, however, according to the present invention it is possible to separate directly the mixture resulting from the resolution.

As already seen, not only the compounds of formula (I) or formula (IX) enantiomerically enriched, or their salts, but also the compounds of formula (II) or formula (X) enantiomerically enriched, or its enantiomers or salts thereof allow to access to enantiomerically enriched *trans-*ciclopentane-1,2-diamine and/or its enantiomer, and to the *meso cis-*ciclopentane-1,2-diamine, or salts thereof, by cleavage (deprotection) of the carbamate group of compounds of formula (I) or formula (IX); and/or the sequential or simultaneous cleavage of the acetate and carbamate groups of the compounds of formula (II) or formula (X), preferably by acid or basic hydrolysis.

Applicable conditions to the present invention can be found in Tetrahedron: Asymm. 2008, 19, 751-755 (see scheme 3 on page 753) or J. Org. Chem. 2007, 72, 1309 (see scheme 3 on page 1313), which are entirely incorporated to the present invention. General methods for deprotection of amino groups can also be found in Greene, T. W.; Wuts, P. G. M. Greene's Protective Groups in Organic Synthesis, John Wiley & Sons: Hoboken, 2007.

According to a preferred embodiment, the hydrolysis of compounds of formula (I) or (II) enantiomerically enriched is acid, preferably with hydrochloric acid, obtaining both enantiomers of the dichloride salt of *trans-*cyclopentane-1,2-diamine: (1*S*,2*S*)**-10** or (1*R*,2*R*)**-10** (see scheme 6).

Similarly, a preferred embodiment is the acid hydrolysis of the compounds of formula (IX) or (X) enantiomerically enriched.

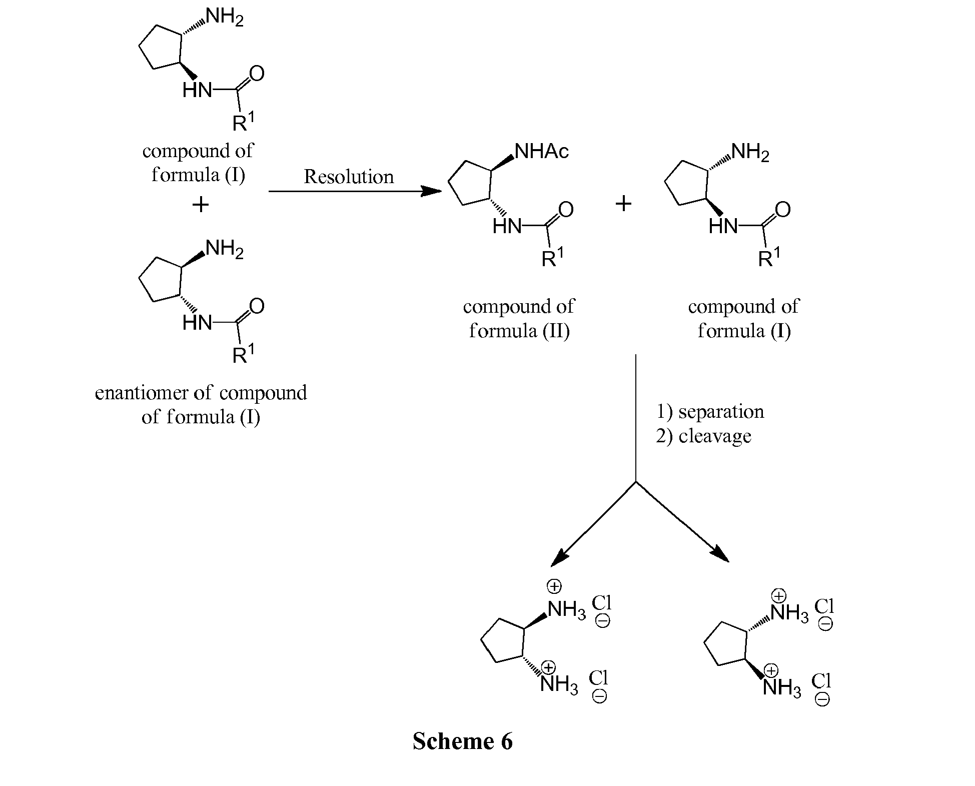

Preferably, the preparation is carried out from the compounds (1*S*,2*S*)**-5**, (1*R*,2*R*)**-7** enantiomerically enriched, preferably enantiomerically pure; or from the compounds (1*S*,2*S*)**-6**, (1*R*,2*R*)**-8** enantiomerically enriched, preferably enantiomerically pure (see scheme 7).

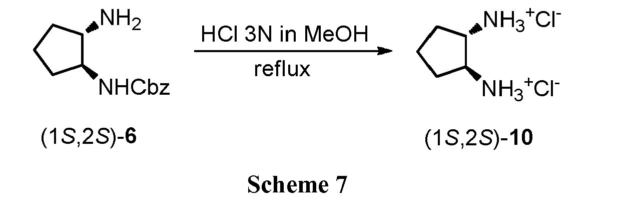

One aspect of the present invention relates to a process for preparing an enantiomerically enriched compound of formula (I), its enantiomers, or salts thereof which comprises carrying a monocarbamoylation reaction in the presence of *trans-*cyclopentane-1,2-diamine, its enantiomers, or salts thereof at a temperature between -30 °C to 50°C, preferably between -20°C to 40 °C, more preferably between -10°C to 30 °C and even more preferably between -10°C and room temperature. Preferably, the reaction procedure consists of the slow addition of one equivalent of a base, preferably triethylamine to a solution of enantiomerically enriched *trans-*cyclopentane-1,2-diamine or salts thereof, preferably its dihydrochloride salt (compounds (1*S*,2*S*)**-10** or (1*R*,2*R*)-**10**), and one equivalent of the corresponding precursor of the carbamate. Reactions for the formation of carbamates are well known to those skilled in the art (for example, see Greene, T. W.; Wuts, P. G. M. Greene's Protective Groups in Organic Synthesis, John Wiley & Sons: Hoboken, 2007). This procedure allows to access to the enantiomer of the compound of formula (I) which was acylated in the resolution process of the invention. Similarly, another aspect of the invention is a procedure for preparing a compound of formula (IX), or salts thereof, which comprises performing a monocarbamoylation reaction in the presence of *meso cis-*cyclopentane-1,2-diamine or a salt thereof, at a temperature between -30°C to 50 °C, preferably between -20 and 40 °C, more preferably between -10° C to 30° C, more preferably between -10 °C and room temperature

A further aspect of the present invention relates to implementing the above procedure to a racemic or enantiomerically enriched compound of formula (XI), its enantiomers, or salts thereof which comprises carrying a monocarbamoylation reaction in the presence of a diamine, its enantiomer, or salts thereof, at a temperature between - 30 °C to 50 °C, preferably between -20 °C to 40 °C, more preferably between -10 °C to 30 °C and even more preferably between -10 °C and room temperature.

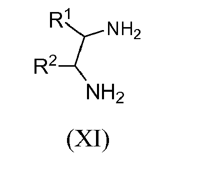

For a better understanding of the procedure object of the present invention, the following examples are provided, which should be understood without limiting character with respect to the scope of the invention.

### EXAMPLES

### Example 1

### Synthesis of the racemic diamine (±)-trans-2.

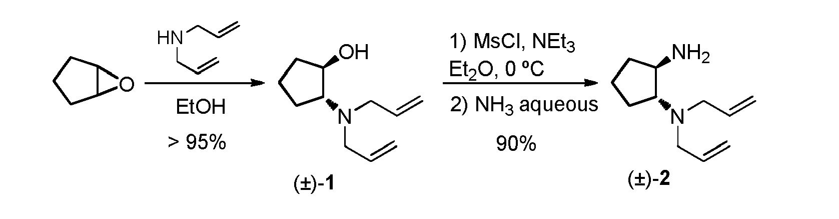

To a solution of 20 mmol of cyclopentene oxide in 40 ml of ethanol is added 60 mmol of diallylamine and the mixture is stirred at 95 ° C for two nights in a sealed tube Subsequently, the solvent and the excess of diallylamine is removed by evaporation at reduced pressure and the aminoalcohol *trans* (±)**-1** is obtained in state of purity in quantitative yield. The above product is dissolved in 80 ml of anhydrous diethyl ether and 64 mmol of dry triethylamine are added. Then, the above solution is cooled to 0 °C and 48 mmol of mesyl chloride are added slowly with vigorous stirring. Once completed the addition, the stirring is maintained at that temperature for 30 minutes. After that time, 80 mmol of dry triethylamine are added, 100 ml of concentrated aqueous ammonia and the resulting biphasic system is stirred for 16 hours at room temperature. The phases are separated and the aqueous phase is extracted twice with 60 ml of diethyl ether. The combined organic phases are treated with anhydrous Na₂SO₄, filtered and concentrated to dryness. The product *trans* (±)**-2** is obtained, after purification by distillation under reduced pressure, with an overall yield of 90%. Colorless liquid, bp 53-55 ° C (0.5 Torr).
¹H-RMN (CDCl₃), δ (ppm): 1.02-1.83 (m, 6H), 1.85 (brs, 2H), 2.64 (q, 1H, *J* 7.8 Hz), 2.85 (dd, 2H, *J* 7.2 y 14.3 Hz), 2.97 (q, 1H, *J* 8.1 Hz), 3.15 (dd, 2H, *J* 5.1 y 14.3 Hz), 5.00 (m, 4H), 5.75 (dddd, 2H, *J*5.1, 7.2, 10.2 y 17.3 Hz)
¹³C-RMN (CDCl₃), δ (ppm): 20.56 (CH₂), 23.07 (CH₂), 32.70 (CH₂), 53.68 (CH₂), 54.20 (CH), 70.42 (CH), 116.28 (CH₂), 136.95 (CH).
E.M.-ESI⁺: 181 [(M+H)⁺,100].

### Example 2

### Synthesis of the racemic aminocarbamate trans (±)-5.

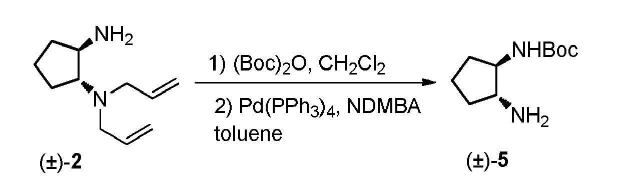

To a solution of 15 mmol of racemic diamine *trans* (±)-2 in 30 ml of dichloromethane is added, slowly and at 0 °C, 16 mmol of di-*tert-*butyl dicarbonate. The resulting mixture was stirred for 7 hours allowing it to reach room temperature. The solvent was evaporated under reduced pressure and the resulting crude product *trans* (±)-**3** is submitted directly to the further step of desallylation without further purification. For its characterization, an analytical sample of (±)**-3** is purified: White solid, mp 53-54 °C.
¹H-RMN (CDCl₃), δ (ppm): 1.28-1.83 (m, 14H), 2.06 (m, 1H), 2.90 (quintet, 1H, *J* 8.4 Hz), 3.15 (dd, 2H, *J* 6.8 y 14.3 Hz), 3.20 (dd, 2H, *J* 5.9 y 14.3 Hz), 3.74 (m, 1H), 4.55 (brs, 1H), 5.08 (dd, 2H, *J* 1.4 y 8.2 Hz), 5.11 (dd, 2H, *J* 4.1 y 17.2 Hz), 5.85 (dddd, 2H, *J* 5.1, 7.2, 10.1 y 17.2 Hz).
¹³C-RMN (CDCl₃), δ (ppm): 20.05 (CH₂), 23.02 (CH₂), 30.41 (CH₃), 33.78 (CH₂), 50.96 (CH), 55.48 (CH₂), 69.27 (CH), 78.01 (C), 118.79 (CH₂), 138.66 (CH), 155.62 (C).
E.M.-ESI+: 281 [(M+H)+,100].

To a solution of 0.3 mmol of Pd(PPh₃)₄ in 15 mmol of dichloromethane are added, under nitrogen atmosphere, a solution of *trans* (±)**-3** in 25 ml of dichloromethane and 45 mmol of 1,3-dimethylbarbituric acid. The resulting mixture is stirred at 35 ° C for 7 hours. After that time the solvent is removed under reduced pressure and 50 ml of toluene and 80 ml of 1N NaOH are added to the resulting crude. The phases are separated and the organic phase is washed twice with 30 ml of water and one with 30 ml of brine. Then the organic phase is extracted three times with 20 ml of a 50% acetic acid aqueous solution. The acidic aqueous phases are collected and 10N NaOH is slowly added at 0 °C. until the solution reaches a pH above 12. The resulting basic solution is extracted three times with 50 ml of diethyl ether. The organic phase is treated with anhydrous Na₂SO₄, filtered and concentrated to dryness. The product *trans* (±)**-5** is obtained with a yield of 85%. White solid mp 60-62 °C.
¹H-RMN (CDCl₃), δ (ppm): 1.10-1.65 (m, 15H), 1.75-2.05 (m, 2H), 2.85 (q, 1H, *J* 7.3 Hz), 3.35-3.45 (m, 1H), 4.44 (brs, 1H, NH).
¹³C-RMN (CDCl₃), δ (ppm): 20.71 (CH₂), 28.33 (CH₃), 31.04 (CH₂), 33.01 (CH₂), 59.54 (CH), 60.42 (CH), 79.18 (C), 155.01 (C).
E.M.-ESI⁺: 201 [(M+H)⁺,100].

### Example 3

### Synthesis of the racemic aminocarbamate trans (±)-6.

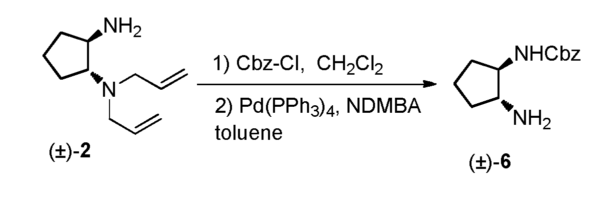

To a solution of 15 mmol of racemic diamine *trans* (±)**-2** in 30 ml of dichloromethane is added, slowly and at 0 °C, 16 mmol of benzyloxycarbonyl chloride. The resulting mixture is stirred for 7 hours allowing it to reach room temperature. After that time the organic phase is washed successively with 30 ml of 1N NaOH and 30 ml of brine. Then, is treated with anhydrous Na₂SO₄, filtered and concentrated to dryness. The resulting crude product *trans* (±)**-4** is submitted directly to the subsequent deallylation step without further purification and following an analogous procedure to that described for the synthesis of *trans* (±)**-5** in the example 2. For its characterization, an analytical sample of (±)**-4** is purified: Colorless oil.
¹H-RMN (CDCl₃), δ (ppm): 1.23-1.80 (m, 5H), 2.11 (quintet, 1H, *J* 6.2 Hz), 2.84-2.97 (m, 1H), 3.04 (dd, 2H, *J* 6.9 y 14.3 Hz), 3.21 (dd, 2H, *J* 5.8 y 14.3 Hz), 3 . 81 (m, 1H), 4.82 (brs, 1H), 5.07 (m, 6H), 5.85 (dddd, 2H, *J* 5.8, 7.2, 10.1 y 17.2 Hz)
¹³C-RMN (CDCl₃), δ (ppm): 20.70 (CH₂), 23.91 (CH₂), 31.29 (CH₂), 53.32 (2 x CH₂), 53.40 (CH), 66.28 (CH₂), 66.94 (CH), 116.70 (CH₂), 127.79 (CH), 128.33 (CH), 136.40 (CH), 156.02 (C).
E.M.-ESI⁺: 315 [(M+H)⁺,100].

Finally, the product *trans* (±)**-6** is obtained with a yield of 82%. Colorless oil. ¹H-RMN (CDCl₃), δ (ppm): 1.25-1.40 (m, 2H), 1.55-1.80 (m, 4H), 1.90-2.00 (m, 1H), 2.05-2.20 (m, 1H), 2.99 (q, 1H, *J* 7.3 Hz), 3.57 (m, 1H), 4.87 (brs, 1H, NH), 5.08 (s, 2H), 7.25-7.40 (m, 5H, Ph).
¹³C-RMN (CDCl₃), δ (ppm): 20.54 (CH₂), 30.89 (CH₂), 32.95 (CH₂), 59.87 (CH), 60.70 (CH), 66.59 (CH₂), 128.34 (CH), 128.43 (CH), 136.36 (C), 156.41 (C).
E.M.-ESI⁺: 235 [(M+H)⁺,100].

### Example 4

### Resolution by enzymatic acylation of the racemic aminocarbamate trans (±)-5.

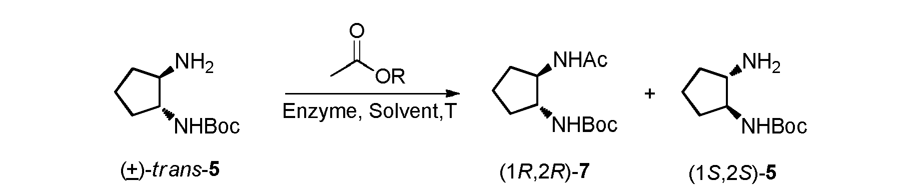

To a flask containing 10.0 mmol of *tert-*butyl *trans* (±)-*N-*(2-aminocyclopentyl) carbamate (**5**) and CALB (1.0 g) is added, under nitrogen atmosphere, 60.0 ml of ethyl acetate. The resulting mixture was stirred at 10 °C and 200 rpm. When the desired conversion is reached (depending on whether enantiopure substrate or product is desired), the enzyme is filtered and washed with methanol. The solvents of the filtrate are evaporated under reduced pressure and the crude is redissolved in 40 ml of dichloromethane. The organic phase is extracted twice with 30 ml of 3N HCl, treated with anhydrous Na₂SO₄, filtered and concentrated to dryness giving rise to the product of this enzymatic reaction, the *tert-*butyl (1*R*,2*R*)-*N-*[2-(acetylamino)cyclopentyl)] carbamate (7). Yield 90%. Conversion: 49%, ee > 98%. The product is recrystallized from ethanol and obtained in enantiomerically pure form. White solid mp 162-164 ° C. ee >99%, [α]_{D}²⁰ +38.2 (c 0.67, CHCl₃).
¹H-RMN (CDCl₃), δ (ppm): 1.30-1.45 (m, 11H), 1.60-1.75 (m, 2H), 1.94 (s, 3H), 2.00-2.20 (m, 2H), 3.65-3.90 (m, 2H), 4.89 (brs, 1H, NH), 6.29 (brs, 1H, NH).
¹³C-RMN (CDCl₃), δ (ppm): 19.40 (CH₂), 23.19 (CH₃), 28.23 (CH₃), 29.15 (CH₂), 29.70 (CH₂), 56.76 (CH), 57.43 (CH), 79.57 (C), 155.63 (C), 170.89 (C).
E.M.-ESI⁺: 243 [(M+H)⁺,65].

The acidic aqueous phases are pooled and 3N NaOH is slowly added at 0 ° C until the solution reaches a pH above 12. The resulting basic solution is extracted three times with 30 ml of diethyl ether. The organic phase is treated with anhydrous Na₂SO₄, filtered and concentrated to dryness yielding the remaining substrate of the enzymatic reaction, the *tert-*butyl (1*S*,2*S*)-*N-*(2-aminocyclopentyl) carbamate (**5**) with a yield of 90%. White solid, mp 60-62 °C. ee >99%, [α]_{D}²⁰ +10.4 (c 1.0, CDCl₃).
¹H-RMN (CDCl₃), δ (ppm): 1.10-1.65 (m, 15H), 1.75-2.05 (m, 2H), 2.85 (q, 1H *J* 7.3 Hz), 3.35-3.45 (m, 1H), 4.44 (brs, 1H, NH).
¹³C-RMN (CDCl₃), δ (ppm): 20.7 (CH₂), 28.3 (CH₃), 31.0 (CH₂), 33.0 (CH₂), 59.5 (CH), 60.4 (CH), 79.2 (C), 156.1 (C).
E.M.-ESI⁺: 201 [(M+H)⁺, 100].

### Example 5

### Resolution by enzymatic acylation of the racemic aminocarbamate trans (±)-6.

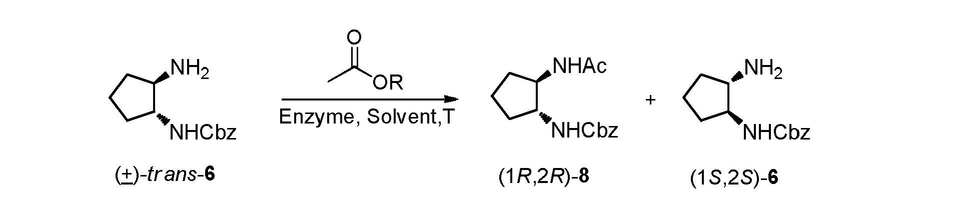

An analogous procedure to that discussed for aminocarbamate *trans* (±)**-5** described in the example 4 is followed. In this way, benzyl (1*R*,2*R*)-*N-*[2-(acetylamino)cyclopentyl)] carbamate (**8**) with a yield of 90%. That product is recrystallized from ethanol and obtained in enantiomerically pure form. White solid mp 200-202 °C. ee >99%, [α]_{D}²⁰ +23.3 (c 0.67, CHCl₃).
¹H-RMN (CDCl₃), δ (ppm): 1.20-1.40 (m, 2H), 1.55-1.70 (m, 2H), 1.84 (s, 3H), 2.00-2.20 (m, 2H), 3.64 (m, 1H), 3.81 (m, 1H), 4.99 (s, 2H), 5.19 (d, 1H, J 6.2 Hz, NH), 5.99 (brs, 1H, NH), 7.10-7.30 (5H, Ph).
¹³C-RMN (CDCl₃), δ (ppm): 19.49 (CH₂), 23.13 (CH₃), 29.57 (2 x CH₂), 56.73 (CH), 57.67 (CH), 66.59 (CH₂), 127.86 (CH), 127.97 (CH), 128.37 (CH), 136.32 (C), 156.92 (C) 170.96 (C).
E.M.-ESI⁺: 277 [(M+H)⁺,100], 299 [(M+Na)⁺,65].

The remaining substrate of this enzymatic reaction, the benzyl (1*S*,2*S*)-N-(2-aminocyclopentyl)carbamate (**6**) is obtained with a yield of 90%. Colorless oil, ee >99%; [α]_{D}²⁰ +16.5 (c 0.5, CHCl₃).
¹H-RMN (CDCl₃), δ (ppm): 1.23-1.49 (m, 2H), 1.53-1.84 (m, 2H), 1.87-2.24 (m, 4H), 3.00 (q, 1H, *J* 7.3 Hz), 3.56 (quintet, 1H, *J* 7.2 Hz), 5.50 (d, 1H, *J* 5.0 Hz, NH), 5.7 (s, 2H, O-C*H*₂-Ph), 7.24-7.43 (m, 5H, Ph).
¹³C-RMN (CDCl₃), δ (ppm): 20.5 (CH₂), 30.8 (CH₂), 32.7 (CH₂), 59.2 (CH), 60.5 (CH), 66.6 (CH₂), 116.7 (CH), 127.9 (CH), 128.4 (CH), 136.3 (C), 156.4 (C).
E.M.-ESI⁺: 235 [(M+H)⁺, 100].

### Example 6

### Synthesis of tert-butyl (1R,2R)-N-(2-aminocyclopentyl)carbamate, (1R,2R)-5

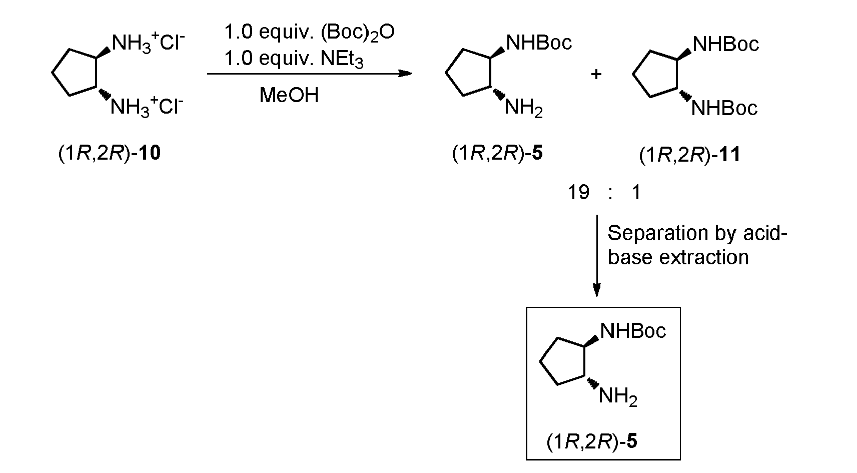

To a solution of 5.0 mmol of (1*R*,2*R*)**-10** and 5.0 mmol of Boc anhydride in MeOH (80 ml) is dropped, slowly and at 0 °C, 5.0 mmol of triethylamine (about 15 minutes with compensated addition funnel). The reaction is stirred at that temperature for 24 hours. The solvent is evaporated under reduced pressure and 150 ml of CH₂C1₂ and 150 ml of 1N NaOH is added to the resulting oil. The phases are separated and the aqueous phase is discarded. The organic phase is extracted three times with 100 ml of 3N HCl. The acidic aqueous phases are pooled and 3N NaOH is slowly added at 0 ° C until the solution reaches a pH above 12. The resulting basic solution is extracted three times with 75 ml of diethyl ether. The organic phase is treated with anhydrous Na₂SO₄, filtered and concentrated to dryness. Yield 75%. White solid, ee >99%, [α]_{D}²⁰ -10.6 (c 1.0, CHCl₃).

### Example 7

### Resolution by enzymatic acylation of the aminocarbamate cis (±)-15.

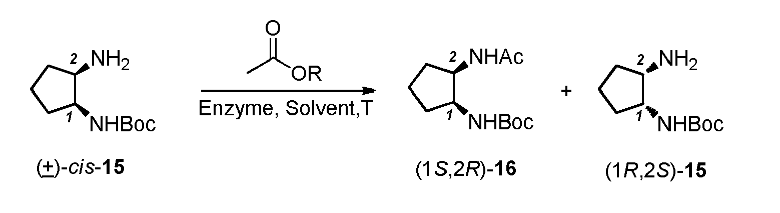

An analogous procedure to that discussed for the aminocarbamate *trans* (±)-**5** described in the example 4 is followed. In this way, *tert-*butyl (1*S*,2*R*)-*N-*[2-(acetylamino)cyclopentyl)] carbamate (**16**) with a yield of 90%. That product is recrystallized from ethanol and obtained in enantiomerically pure form. White solid mp 118-120 °C. ee >99%, [α]_{D}²⁰ +10.8 (c 1.0, CHCl₃).
¹H-RMN (CDCl₃), δ (ppm): 1.44 (s, 9H), 1.50-1.80 (m, 4H), 1.96 (s, 3H), 2.00-2.20 (m, 2H), 3.92 (m, 1H), 4.15 (m, 1H), 4.76 (brs, 1H, NH), 6.10 (brs, 1H, NH).
¹³C-RMN (CDCl₃), δ (ppm): 20.05 (CH₂), 23.09 (CH₃), 28.17 (CH₃), 29.45 (CH₂), 29.84 (CH₂), 52.93 (CH), 53.45 (CH), 79.21 (C), 156.16 (C) 170.37 (C).
E.M.-ESI⁺: 243 [(M+H)⁺,40].

The remaining substrate of this enzymatic reaction, *tert-*butyl (1*R*,2*S*)-*N-*(2-aminocyclopentyl)carbamate (**15**) is obtained with a yield of 90%. Colorless oil, ee >99%.
¹H-RMN (CDCl₃), δ (ppm): 1.39 (s, 9H), 1.45-1.55 (m, 2H), 1.69 (m, 1H), 1.80-2.00 (m, 3H), 2.23 (brs, 2H, NH₂), 3.29 (q, 1H, *J* 5.5 Hz), 3.76 (m, 1H), 5.14 (brs, 1H, NH). ¹³C-RMN (CDCl₃), δ (ppm): 20.2 (CH₂), 28.2 (CH₃), 29.7 (CH₂), 32.4 (CH₂), 53.0 (CH), 54.6 (CH), 78.9 (C), 155.8 (C).
E.M.-ESI⁺: 201 [(M+H)⁺, 100].

### Example 8

### Synthesis of racemic tert-butyl cis-N-(2-aminocyclopentyl)carbamate, (±)-15.

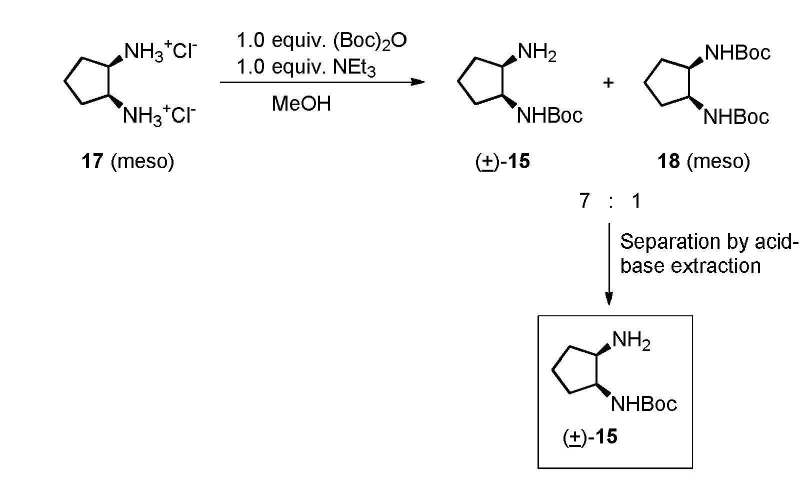

Starting from the dihydrochloride of *cis-*cyclopentane-1,2-diamine (**17**) and following the protocol described in the example 6 is possible to prepare the corresponding racemic monocarbamate (±)**-15** with a yield of 65%. Regarding the initial dihydrochloride 17, it can be prepared according to Tetrahedron 2002, 58, 1131.

## Claims

**1.** A procedure for
(A) obtaining a mixture comprising an enantiomerically enriched compound of formula (I), or salts thereof, and an enantiomerically enriched compound of formula (II), or salts thereof, or to obtain a mixture comprising the enantiomerically enriched enantiomer of the compound of formula (I), or salts thereof, and the enantiomerically enriched enantiomer of the compound of formula (II), or salts thereof, 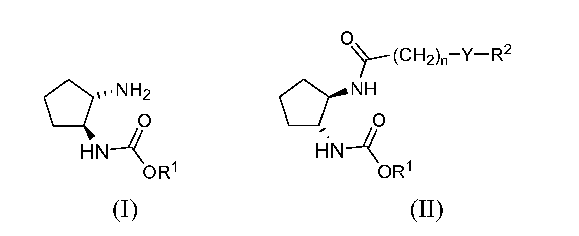 or
(B) obtaining a mixture comprising an enantiomerically enriched compound of formula (IX), or salts thereof, and an enantiomerically enriched compound of formula (X), or salts thereof, or to obtain a mixture comprising the enantiomerically enriched enantiomer of the compound of formula (IX), or salts thereof, and the enantiomerically enriched enantiomer of the compound of formula (X), or salts thereof, 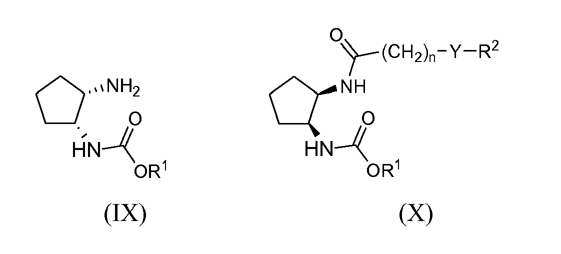 where
R¹ is selected from the group consisting of substituted or unsubstituted alkyl, substituted or unsubstituted alkenyl and substituted or unsubstituted arylalkyl;
R² is selected from the group consisting of hydrogen, substituted or unsubstituted alkyl, haloalkyl and unsubstituted or mono-, di- or trisubstituted phenyl with identical or different substituents selected from the group consisting of halogen, amino, hydroxy, C1-C₄ alkyl, C₁-C₄ alkoxy, phenyl and phenoxy;
n represents the numbers 0, 1, 2 or 3, and
Y represents a bond, oxygen, sulfur, or a group -NR⁸ where R⁸ represents hydrogen or a C₁-C₄ alkyl group;
comprising
reacting a mixture which comprises said compound of formula (I) and its enantiomer, or its salts; or
reacting a mixture which comprise said compound of formula (IX) and its enantiomer, or its salts,
with an acylating agent in the presence of an enzyme.

**2.** The procedure according to claim 1, wherein R¹ is selected from the group consisting of *tert-*butyl, benzyl, allyl, methyl, ethyl, *iso*-butyl, *p*-methoxybenzyl and 9-fluorenylethyl.

**3.** The procedure according to claim 1 or 2, wherein -(CH₂)ₙY-R² represents methyl, methoxymethyl, chloromethyl and bromomethyl.

**4.** The procedure according to any of claims 1 to 3, which further comprises separating the compounds (II) and (I) or its enantiomers; or separating the compounds (X) and (IX) or its enantiomers.

**5.** The procedure according to claim 4, wherein the separation step is performed by acid-base extraction, recrystallization or a combination thereof.

**6.** The procedure according to any preceding claim, wherein the enzyme is a lipase.

**7.** The procedure according to any preceding claim, wherein the enzyme is the fraction B of *Candida antarctica* lipase.

**8.** The procedure according to any preceding claim, wherein the enzyme is immobilized on a support.

**9.** The procedure according to claim 8, wherein the support on which the lipase is immobilized is an epoxiacrylic resin activated with deca-octyl groups.

**10.** The procedure according to any preceding claim, where the acylating agent is a compound of general formula (VIII) 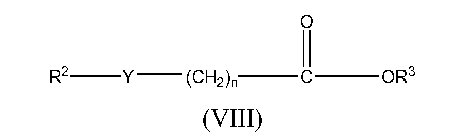 where
R², Y and n are as defined in claim 1; and
R³ is selected from the group consisting of substituted or unsubstituted alkyl and haloalkyl.

**11.** The procedure according to claim 10, wherein the acylating agent is selected from the group consisting of ethyl acetate, isopropyl acetate, methyl methoxyacetate, isopropyl methoxyacetate, ethyl chloroacetate, ethyl bromoacetate, methyl chloroacetate, methyl bromoacetate, α-methylbenzyl acetate, α-methylbenzyl methoxyacetate, α-methylbenzyl chloroacetate and α-methylbenzyl bromoacetate.

**12.** The procedure according to any preceding claim, wherein the reaction is performed in the presence of a solvent selected from the group consisting of ethyl acetate, dioxane, tetrahydrofuran, *tert-*butyl methyl ether, diisopropyl ether, diethyl ether, toluene, hexane, acetonitrile, acetone, 2-propan-2-ol, 2-methylpropan-2-ol, chloroform and dichloromethane.

**13.** The procedure according to any of claims 10 to 12, wherein the acylating agent and the solvent are both ethyl acetate.

**14.** A mixture comprising
(A) an enantiomerically enriched compound of formula (I), or salts thereof, and an enantiomerically enriched compound of formula (II), or salts thereof, or a mixture comprising an enantiomerically enriched enantiomer of the compound of formula (I), or salts thereof, and the enantiomerically enriched enantiomer of the compound of formula (II), or salts thereof, 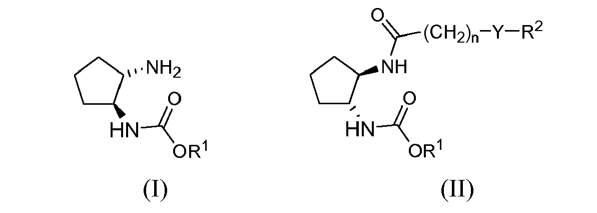 or
(B) an enantiomerically enriched compound of formula (IX), or salts thereof, and an enantiomerically enriched compound of formula (X), or salts thereof, or the enantiomerically enriched enantiomer of the compound of formula (IX), or salts thereof, and the enantiomerically enriched enantiomer of the compound of formula (X), or salts thereof, 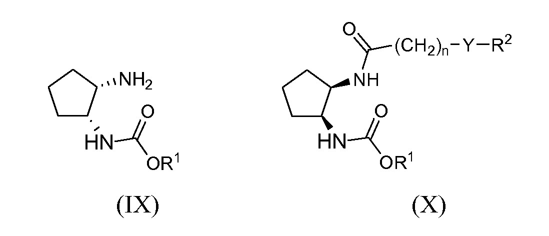 where R¹, R², Y and n are as defined in claim 1.

**15.** Mixture according to claim 14, wherein R¹ is selected from the group consisting of *tert-*butyl, benzyl, allyl, methyl, ethyl, *iso*-butyl, *p*-methoxybenzyl and 9-fluorenylethyl.

**16.** Mixture according to claim 14 or 15, wherein -(CH₂)ₙY-R² represents methyl, methoxymethyl, chloromethyl and bromomethyl.

**17.** A mixture, which is racemic, according to claims 14 to 16.

**18.** A procedure for the separation of a mixture in their enantiomerically enriched compounds according to claim 14, which comprises an acid-base extraction, recrystallization or a combination of both.

**19.** An enantiomerically enriched compound of formula (II) or formula (X), or its enantiomers or salts thereof 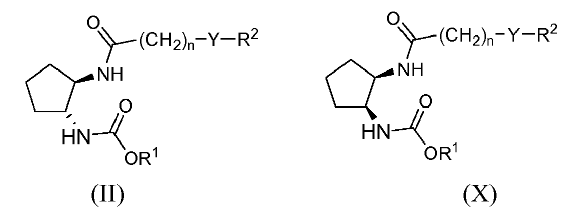 where R¹, R², Y and n are defined as in claim 1.

**20.** Compound according to claim 19, wherein R¹ is selected from the group consisting of *tert-*butyl, benzyl, allyl, methyl, ethyl, *iso*-butyl, *p*-methoxybenzyl and 9-fluorenylethyl.

**21.** Compound according to claim 19 or 20, wherein - (CH₂)ₙY-R² represents methyl, methoxymethyl, chloromethyl and bromomethyl.

**22.** A procedure for obtaining enantiomerically enriched *trans-*cyclopentane-1,2-diamine or *meso cis-*cyclopentane-1,2-diamine, or salts thereof comprising the following steps:
a) the procedure defined in any one of claims 1 to 13;
b) separating the mixture obtained in the previous step in the enantiomerically enriched compounds (II) and (I) or enantiomerically enriched compounds (X) and (IX), or their enantiomers, preferably by acid/base extraction, recrystallization or a combination of both, and
c) cleaving the carbamate group of that compound of formula (I) or formula (IX) and/or sequentially or simultaneously splitting the groups -(C=O)-(CH₂)ₙY-R² and carbamate of the compound of formula (II) or of the compound of formula (X), preferably by acid or basic hydrolysis.

**23.** Use of a mixture comprising a compound of formula (I) and its enantiomer or salts thereof to obtain enantiomerically enriched *trans-*cyclopentane-1,2-diamine, or salts thereof.

**24.** Procedure for the synthesis of a compound of formula (VI), or its enantiomerically enriched enantiomer, 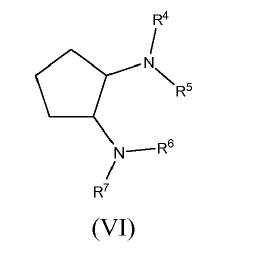 where
R⁴, R⁵, R⁶ and R⁷ are independently selected from the group consisting of hydrogen, substituted or unsubstituted alkyl, substituted or unsubstituted cycloalkyl, aromatic or nonaromatic, substituted or unsubstituted heterocyclic, substituted or unsubstituted hydroxyalkyl, haloalkyl, substituted or unsubstituted alkoxyalkyl, substituted or unsubstituted alkenyl, substituted or unsubstituted alkynyl, substituted or unsubstituted aryl, substituted or unsubstituted alkylaryl, COOR, carbonate, -COOH, -COH, -COR, - (C=O)NH₂, -(C=O)NRₐR_{b}, silyl group, -S(=O)R or a combination of them; and where R and Ra and Rb are independently selected from a C₁-C₈ alkyl group;
comprising the preparation of enantiomerically enriched *trans-*cyclopentane-1,2-diamine or *meso cis-*cyclopentane-1,2-diamine, or its salts, according to the procedure defined in claim 22; and the functionalization of the amino groups for the introduction of the groups R⁴, R⁵, R⁶ and R⁷.

**25.** Use of a mixture comprising a compound of formula (I) and/or its enantiomers or salts thereof, or a compound of formula (IX) and/or its enantiomers or salts thereof, to obtain a compound of formula (VI) and/or its enantiomer, or salts thereof.

**26.** A mixture of the enantiomers of benzyl *trans-N-*(2-aminocyclopentyl)carbamate, or salt thereof, or of the enantiomers of *tert-*butyl *N-*[2-(acetylamino)cyclopentyl)]carbamate or salts thereof.

**27.** Procedure for the preparation of a racemic mixture of a compound of formula (I) and its enantiomer, or its salts, comprising removing the allyl groups, preferably in the presence of palladium, of the racemic mixture of a compound of formula (VII) and its enantiomer 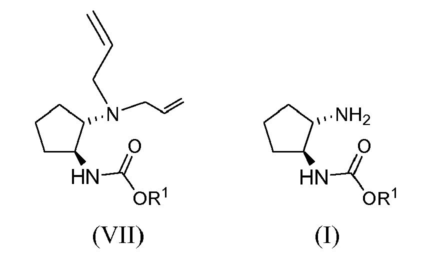 where R¹ is selected from substituted or unsubstituted alkyl, substituted or unsubstituted alkenyl and substituted or unsubstituted arylalkyl.

**28.** Procedure according to claim 27, wherein said racemic mixture of a compound of formula (VII) and its enantiomer is prepared by carbamoylation of *trans-*(±)-*N,N-*diallylcyclopentane-1,2-diamine in the presence of a carbamoylation agent.

**30.** A procedure for preparing an enantiomerically enriched compound of formula (I), its enantiomers, or salts thereof; or for the preparation of a racemic compound of formula (IX), or salts thereof 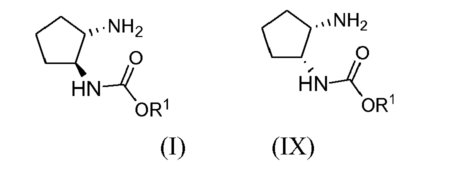 where R¹ is selected from substituted or unsubstituted alkyl, substituted or unsubstituted alkenyl and substituted or unsubstituted arylalkyl;
which comprises carrying out a reaction of monocarbamoylation in the presence of enantiomerically enriched *trans-*cyclopentane-1,2-diamine, or salts thereof, or *meso cis-*cyclopentane-1,2-diamine, respectively, at a temperature between -30 °C and 50 °C, preferably between -20 and 40 °C, more preferably between -10 °C and 30 °C, more preferably between -10 °C and room temperature.

**31.** A compound of formula (**6**), or enantiómeros thereof

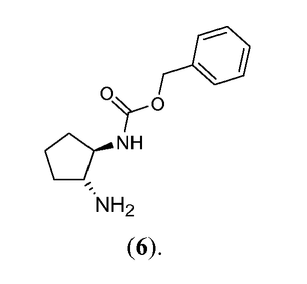

**32.** A mixture of a compound of formula (VII) and enantiómeros thereof, or salts thereof 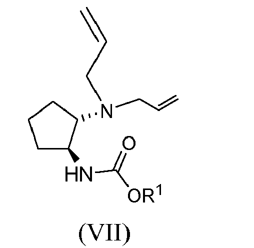 wherein R¹ is selected from substituted or unsubstituted alkyl, substituted or unsubstituted alkenyl and substituted or unsubstituted arylalkyl with the proviso that R¹ is not *tert-*butyl.

**33.** A process for preparing a racemic or enantiomerically enriched compound of formula (XI), its enantiomers, or salts thereof 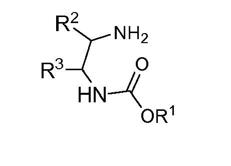
where R¹ is selected from substituted or unsubstituted alkyl, substituted or unsubstituted alkenyl and substituted or unsubstituted arylalkyl; and
the diamine (R², R³) is selected from the group consisting of *trans-*cyclohexane-1,2-diamine, *cis-*cyclohexane-1,2-diamine, *trans-*cyclopentane-1,2-diamine, *cis-*cyclopentane-1,2-diamine, *trans-*1,2-diphenyl-1,2-ethylenediamine, ethylene-1,2-diamine, propane-1,3-diamine and 2-hydroxypropane-1,3-diamine
comprising a monocarbamoylation reaction in the presence of the corresponding racemic or enantiomerically enriched diamine, or salts thereof, at a temperature between -30 °C and 50 °C, preferably between -20 and 40 ° C, more preferably between -10 °C and 30 °C, more preferably between -10 °C and room temperature.
